# EUROPEAN PATENT APPLICATION

(11) **EP 4 478 048 A1**
(43) Date of publication of application: **18.12.2024**
(21) Application number: 24181965.5
(22) Date of filing: 13.06.2024
(51) Int. Cl.: G01N 33/00, F24F 110/64, F24F 110/66, F24F 110/70

(54) **SYSTEM AND METHOD FOR CALIBRATING INDOOR AIR QUALITY SENSORS OF A MULTI-ZONE ENVIRONMENT**

(30) Priority: 16.06.2023 US 202363508579 P
(71) Applicant: Carrier Corporation, Palm Beach Gardens, FL 33418 (US)
(72) Inventor: SMOOT, David, East Syracuse, 13057 (US); KERLING, Jacob, Minneapolis, 55438 (US); RANJAN, Rajiv, Florida, 33412 (US); CULP, Slade, Bradenton, 34202 (US); BAILEY, Callum, Bradenton, 34202 (US); IVANOV, Stanislav, Bradenton, 34202 (US)
(74) Representative: Dehns

(57) **Abstract**

A method 200 for calibrating indoor air quality (IAQ) sensors associated with an area of interest (AOI) is disclosed. The method comprises the steps of creating a predetermined calibration environment for one or more IAQ sensors associated with the AOI 202 by enabling a building management system (BMS) associated with the AOI to perform one or more of adjusting inflow of outside air into the AOI to a first predetermined maximum level, adjusting ventilation rate of the AOI to a second predetermined maximum level, and increasing a degree of air filtration in the AOI to a third predetermined maximum level; monitoring IAQ values 204 detected by the one or more IAQ sensors at the AOI in the predetermined calibration environment, and calibrating the one or more IAQ sensors 206 based on an average value of the monitored IAQ values being detected by each of the IAQ sensors.

## Description

### BACKGROUND

This invention relates to the field of indoor air quality sensor calibration, and more particularly, a system and method for calibrating indoor air quality (IAQ) sensors associated with a multizone environment or an area of interest.

In a multi-zone environment, different IAQ sensors may be employed at each zone, which may allow users to monitor the IAQ of the respective zones. IAQ sensors are to be calibrated periodically or annually to effectively operate and meet industrial and environmental standards. IAQ sensors may require a predictable environment in the multi-zone environment (where they are installed) as a calibration baseline to perform the calibration. However, some sensors may not follow a predictable cycle and, as a result, this passive automatic baseline calibration may not be reliable and effective.

### SUMMARY

Described herein is a method for calibrating indoor air quality (IAQ) sensors associated with an area of interest (AOI). The method comprises the steps of creating a predetermined calibration environment for one or more IAQ sensors associated with the AOI; monitoring IAQ values detected by the one or more IAQ sensors at the AOI in the predetermined calibration environment at a predefined time, and calibrating the one or more IAQ sensors based on the monitored IAQ values being detected by each of the IAQ sensors.

In one or more embodiments, the method may comprise the step of calibrating the one or more IAQ sensors based on an average value of the monitored IAQ values being detected by each of the IAQ sensors.

In one or more embodiments, the method of creating the predetermined calibration environment may comprise the steps of enabling a building management system (BMS) associated with the AOI to perform one or more of adjusting inflow of outside air into the AOI to a first predetermined maximum level, adjusting ventilation rate of the AOI to a second predetermined maximum level, and increasing a degree of air filtration in the AOI to a third predetermined maximum level.

In one or more embodiments, the method may comprise the steps of actuating an air handling unit (AHU) associated with the BMS to adjust inflow of the outside air into the AOI to the first predetermined maximum level, and/or further adjust ventilation rate of the AOI to the second predetermined maximum level; and actuating the AHU or an air filtration system associated with the BMS to increase the degree of air filtration in the AOI to the third predetermined maximum level.

In one or more embodiments, the AOI may be a multi-zone building comprising one or more zones, wherein the method of creating the calibration environment may comprise the steps of actuating air dampers associated with each of the one or more zones and a fan assembly associated with the AHU to adjust inflow of the outside air into the one or more zones to the first predetermined maximum level, and/or further adjust ventilation rate of the one or more zones to the second predetermined maximum level; and actuating the AHU or an air filtration system associated with the BMS to increase the degree of air filtration in the one or more zones to the third predetermined maximum level.

In one or more embodiments, the predetermined calibration environment may be created when no occupant is detected in the AOI.

In one or more embodiments, the predetermined calibration environment may be created during the predefined time comprising night time.

In one or more embodiments, the predetermined calibration environment may be created when temperature, and humidity of the outside air and/or a level of air quality parameters comprising one or more of volatile organic compounds, carbon dioxide, particulate matter, carbon monoxide, ethylene, formaldehyde, radon, methane, ozone, sulfur dioxide, nitric oxide, oxygen, and nitrous oxide present in the outside air are within predefined threshold ranges, wherein data pertaining to the level of the air quality parameters, the temperature, and the humidity of the outside air is received from one or more secondary sensors positioned outside the AOI and/or from a server.

In one or more embodiments, the one or more secondary sensors may comprise one or more of a temperature sensor, a humidity sensor, a volatile organic compound sensor, a carbon dioxide sensor, a particulate matter sensor, a carbon monoxide sensor, an ethylene sensor, a formaldehyde sensor, a radon sensor, a methane sensor, an ozone sensor, a sulfur dioxide sensor, a nitric oxide sensor, an oxygen sensor, and a nitrous oxide sensor.

Also described herein is a system to calibrate indoor air quality (IAQ) sensors associated with an area of interest (AOI). The system comprises a controller associated with a building management system (BMS) of the AOI, wherein the controller is in communication with one or more IAQ sensors associated with the AOI, wherein the controller is configured to actuate the BMS to create a predetermined calibration environment for the one or more IAQ sensors of the AOI, monitor IAQ values detected by the one or more IAQ sensors at the AOI in the predetermined calibration environment, and calibrate the one or more IAQ sensors based on the monitored IAQ values being detected by each of the IAQ sensors.

In one or more embodiments, the controller may be configured to calibrate the one or more IAQ sensors based on an average value of the monitored IAQ values being detected by each of the IAQ sensors.

In one or more embodiments, the controller may be configured to create the predetermined calibration environment by actuating the BMS to perform one or more of adjusting inflow of outside air into the AOI to a first predetermined maximum level, adjusting ventilation rate of the AOI to a second predetermined maximum level, and increasing a degree of air filtration in the AOI to a third predetermined maximum level.

In one or more embodiments, the controller may be configured to: actuate an air handling unit (AHU) associated with the BMS to adjust inflow of the outside air into the AOI to the first predetermined maximum level, and/or further adjust ventilation rate of the AOI to the second predetermined maximum level; and actuate the AHU or an air filtration system associated with the BMS to increase the degree of air filtration in the AOI to the third predetermined maximum level.

In one or more embodiments, the system may comprise one or more occupancy sensors positioned at the AOI, and in communication with the controller, wherein the one or more occupancy sensors are operable to detect presence of occupants at the AOI, wherein the controller is configured to create predetermined calibration environment when no occupants are detected in the AOI by the one or more occupancy sensors.

In one or more embodiments, the system may comprise one or more secondary sensors comprising one or more of a temperature sensor, a humidity sensor, a volatile organic compound sensor, a carbon dioxide sensor, a particulate matter sensor, a carbon monoxide sensor, an ethylene sensor, a formaldehyde sensor, a radon sensor, a methane sensor, an ozone sensor, a sulfur dioxide sensor, a nitric oxide sensor, an oxygen sensor, and a nitrous oxide sensor, positioned outside the AOI and in communication with the controller, wherein the one or more secondary sensors are operable to (sense) temperature, and humidity of the outside air and/or a level of air quality parameters comprising one or more of volatile organic compound, carbon dioxide, particulate matter, carbon monoxide, ethylene, formaldehyde, radon, methane, ozone, sulfur dioxide, nitric oxide, oxygen, and nitrous oxide present in the outside air, wherein the controller is configured to create the predetermined calibration environment when the monitored level of the air quality parameters, the temperature, and the humidity of the outside air is detected to be within predefined threshold values.

In one or more embodiments, the AOI may be a multi-zone building comprising one or more zones.

In one or more embodiments, the one or more indoor air quality sensors may comprise one or more of a volatile organic compound sensor, a carbon dioxide sensor, a particulate matter sensor, a carbon monoxide sensor, an ethylene sensor, a formaldehyde sensor, a radon sensor, a methane sensor, an oxygen sensor, an ozone sensor, a sulfur dioxide sensor, a nitric oxide, and a nitrous oxide sensor.

Further described herein is a method for creating a calibration environment for indoor air quality (IAQ) sensors associated with an area of interest (AOI), the method comprising the steps of enabling a building management system associated with the AOI to perform one or more of adjusting inflow of outdoor air into the AOI to a first predetermined maximum level, adjusting ventilation rate of the AOI to a second predetermined maximum level, and increasing a degree of air filtration in the AOI to a third predetermined maximum level.

In one or more embodiments, the calibration environment may be created at a predefined time comprising night time and/or when no occupant is detected at the AOI.

In one or more embodiments, the calibration environment may be created when the air quality of the outside air is less than predefined threshold values.

The foregoing summary is illustrative only. In addition to the illustrative aspects, embodiments, and features described above, further aspects, embodiments, features, and techniques of the subject disclosure will become more apparent from the following description taken in conjunction with the drawings.

### BRIEF DESCRIPTION OF THE DRAWINGS

The accompanying drawings are included to provide a further understanding of the subject disclosure and are incorporated in and constitute a part of this specification. The drawings illustrate exemplary embodiments of the subject disclosure and, together with the description, serve to explain the principles of the subject disclosure.

In the drawings, similar components and/or features may have the same reference label. Further, various components of the same type may be distinguished by following the reference label with a second label that distinguishes among the similar components. If only the first reference label is used in the specification, the description is applicable to any one of the similar components having the same first reference label irrespective of the second reference label. Certain exemplary embodiments will now be described in greater detail by way of example only and with reference to the accompanying drawings in which:
FIG. 1 illustrates an exemplary representation of a system implemented in a multi-zone environment/AOI for creating an artificial calibration environment to calibrate the IAQ sensors of the multi-zone environment/AOI;
FIG. 2 illustrates exemplary steps involved in a method for creating an artificial calibration environment to calibrate the IAQ sensors of the multi-zone environment/AOI; and
FIG. 3 illustrates an exemplary view of an air handling unit of the BMS implemented in the system and method of FIG. 1 and FIG. 2, respectively.

### DETAILED DESCRIPTION

The following is a detailed description of embodiments of the disclosure depicted in the accompanying drawings. The embodiments are in such detail as to clearly communicate the disclosure. However, the amount of detail offered is not intended to limit the anticipated variations of embodiments; on the contrary, the intention is to cover all modifications, equivalents, and alternatives falling within the scope of the claims.

Various terms are used herein. To the extent a term used in a claim is not defined below, it should be given the broadest definition persons in the pertinent art have given that term as reflected in printed publications and issued patents at the time of filing.

In the specification, reference may be made to the spatial relationships between various components and to the spatial orientation of various aspects of components as the devices are depicted in the attached drawings. However, as will be recognized by those skilled in the art after a complete reading of the subject disclosure, the components of this invention described herein may be positioned in any desired orientation. Thus, the use of terms such as "above," "below," "upper," "lower," "first", "second" or other like terms to describe a spatial relationship between various components or to describe the spatial orientation of aspects of such components should be understood to describe a relative relationship between the components or a spatial orientation of aspects of such components, respectively, described herein may be oriented in any desired direction.

In a multi-zone environment such as but not limited to a building having multiple zones such as floors and rooms, a building management system (BMS) may be employed to monitor and control the indoor air quality (IAQ) of the environment. The BMS may include multiple HVAC devices installed at different zones for independently conditioning IAQ of each zone, where each HVAC device may be further fluidically connected to an air handling unit (AHU). The BMS may further include dampers to control airflow into and out of the zones and an air filtration system for filtering the air being supplied to the zones. In addition, the BMS may include one or more IAQ sensors assigned to and provided at each zone for monitoring different IAQ parameters of the environment. The IAQ sensors may allow users to monitor the IAQ level to be maintained at the respective zones and also monitor the real-time IAQ readings of the respective zones. These IAQ sensors may include but are not limited to a temperature sensor, a humidity sensor, a carbon monoxide (CO) sensor, a carbon dioxide sensor (CO2), a volatile organic compound (VOC) sensor, a carcinogenic formaldehyde sensor, a particulate matter sensor, an ethylene sensor, a radon sensor, a methane sensor, an ozone sensor, a sulfur dioxide sensor, a nitric oxide sensor, an oxygen sensor, and/or a nitrous oxide sensor.

The IAQ sensors associated with the environment are to be calibrated periodically or annually to effectively operate and meet industrial and governmental standards. These IAQ sensors may need a predictable calibration environment in the multi-zone environment (where they are installed), as a calibration baseline to perform the calibration. For instance, as indoor air quality parameters such as carbon dioxide may fluctuate in a predictable natural cycle, the low value, typically the unoccupied nighttime value may be accepted as a calibration baseline for calibrating the carbon dioxide sensors. However, other sensors such as volatile organic compound sensors, particulate matter sensors, and the like may not follow a predictable cycle, as a result, the automatic calibration baseline for these IAQ sensors may not be reliable and effective.

This invention provides a simple, efficient, and cost-effective solution involving the existing building management system of the multi-zone environment (area of interest) for creating an artificial calibration environment or calibration baseline for the IAQ sensors to calibrate the IAQ sensors of the multi-zone environment, without additional hardware components.

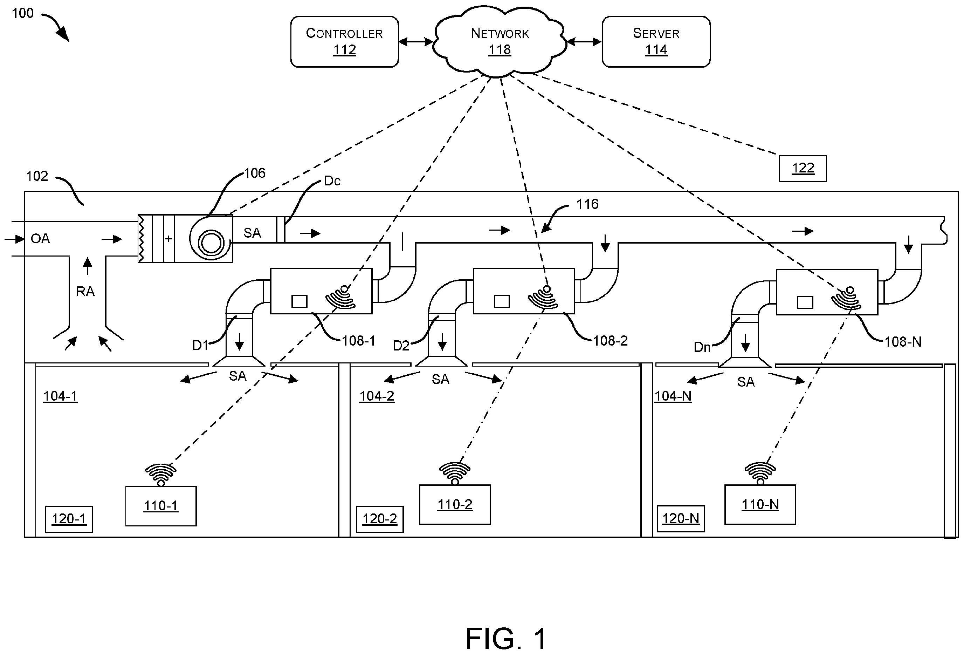

Referring to FIG. 1, a system 100 configured with a building management system (BMS) of a multi-zone environment/area of interest 102 is disclosed, which may enable the creation of an artificial predictable calibration environment or baseline for the indoor air quality (IAQ) sensors 110 of the multi-zone environment/AOI 102 for automatic calibration of the IAQ sensors 110 without any additional hardware components. The BMS may be capable of controlling and preserving the IAQ of the environment/AOI 102. The multi-zone environment/AOI 102 (also referred to as an area of interest (AOI), herein) may include a plurality of zones 104-1 to 104-N (collectively referred to as zones 104, herein). In one or more embodiments, the environment/AOI 102 may be a building and the zones 104 may be the rooms of the building 102. However, in other embodiments, the environment/AOI 102 may be a shipment and the zones 104 may be containers or storage space associated with shipment 102, and all such embodiments are well within the scope of the subject disclosure.

The system 100 or BMS may include one or more HVAC devices 108-1 to 108-N (collectively referred to as HVAC devices 108, herein) configured with the zones 104-1 to 104-N such that at least one HVAC device 104 is assigned to and fluidically connected to a space associated with each of the zones 104. Each of the HVAC devices 108 may be further fluidically connected to an air handling unit 106 installed in the AOI 102. Further, each zone 104-1 to 104-N may include at least one indoor air quality (IAQ) sensor 110-1 to 110-N (collectively referred to as IAQ sensors 110, herein), to monitor the IAQ of the zones 104-1 to 104-N. The IAQ sensors 110 may be configured to communicatively couple or communicate with the HVAC devices 108, a central server 114, and/or a controller 112 associated with the BMS through a network 118. The detailed operation and construction of the BMS and the AHU 106 have been described in the subsequent paragraphs.

As illustrated, in one or more embodiments, HVAC device 108-1 may be assigned to and installed in zone 104-1, and IAQ sensor 110-1 may be assigned to and provided in zone 104-1. Further, HVAC device 108-2 may be assigned to and installed in zone 104-2, and IAQ sensor 110-2 may be assigned to and provided in zone 104-2. Similarly, the N-number of HVAC devices 108-N, and IAQ sensors 110-N may be assigned and installed at the N-number of zones 104-N of the AOI 102. It would be obvious for a person skilled in the art that while various embodiments of the subject disclosure have been elaborated for an AOI having three zones 104-1, 104-2, 104-N, however, teachings of the subject disclosure are applicable to AOI having any number of zones, and all such embodiments are well within the scope of the subject disclosure.

In one or more embodiments, the IAQ sensors 110 employed in the environment/AOI 102 may be a carbon monoxide (CO) sensor, a carbon dioxide sensor (CO2), a volatile organic compound (VOC) sensor, a carcinogenic formaldehyde sensor, particulate matter sensor, an ethylene sensor, a radon sensor, a methane sensor, an ozone sensor, a sulfur dioxide sensor, a nitric oxide sensor, an oxygen sensor, and a nitrous oxide sensor, but not limited to the like. In addition, a temperature sensor and a humidity sensor may be employed in the AOI 102. The captured IAQ data of the zones 104 may then be transmitted to the central server 114 or the controller 112 of BMS or the HVAC devices 108 via a transceiver for further processing, which may further allow the BMS to control the operation of the connected HVAC device 108 based on the user-defined IAQ level set by users in the IAQ sensor 110. In one or more embodiments, different IAQ sensors 110 may be distributed at different positions within the zones 104 of the AOI 102. The IAQ sensors 110 may be operatively connected in a mesh network where a master sensor among the sensors 110 may be in communication with the server 114 or the controller 112 of the BMS via wired media or wireless media.

In some embodiments, the IAQ sensors 110, the temperature sensor, and the humidity sensor may be a single sensing device where the corresponding sensors may be installed in a single housing, which may be provided at each zone of the environment/AOI. The IAQ sensors/sensing device 110 may be operable to monitor the levels of carbon monoxide, carbon dioxide, particulate matter, total volatile compounds, carcinogenic formaldehyde, ethylene, radon, methane, ozone, oxygen, sulfur dioxide, nitric oxide, nitrous oxide, and bacteria, but not limited to the like, present in the zones 104 where the IAQ sensor/sensing device is present. In addition, the IAQ sensors/sensing device 110 may include an input unit such as a keyboard or buttons that may allow users to monitor as well as set the IAQ level to be maintained at the respective zones. Further, the IAQ sensors/sensing device 110 may include a display unit configured to display the monitored IAQ of the space associated with the zones 104, and the IAQ values set by the user. The captured IAQ data of the zones 104 may then be transmitted to the central server 114 and/or the controller 112 and/or HVAC device 108 of the BMS via a transceiver for further processing, which may further allow the BMS to control the operation of the connected HVAC device 108 based on the user-defined IAQ level set by users in the IAQ sensor 110.

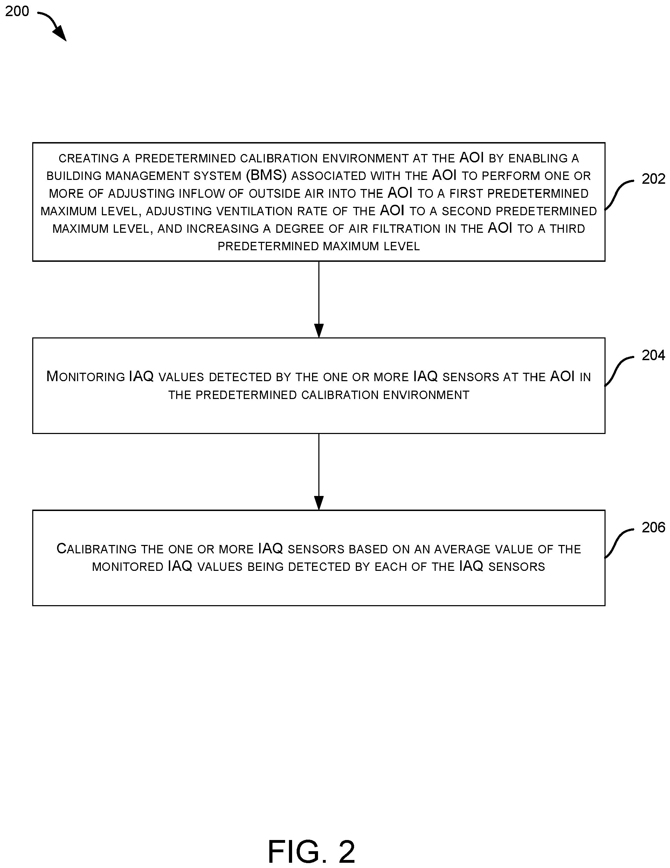

The BMS or AHU 106 may include a duct system 116 comprising a supply air stream duct comprising a plurality of interconnected supply channels fluidically connected to each zone 104 via the HVAC devices 108 to facilitate the inflow of supply air (SA) into the zones 104 of the AOI 102 after getting conditioned by the corresponding HVAC device 108, and a return air duct comprising a plurality of interconnected return channel ducts to facilitate outflow of return air (RA) from each of the zones 104. The HVAC system/BMS or AHU 106 may further include a central damper (Dc) configured to regulate the flow of supply air SA comprising any or a mixture of outside air OA and the return air RA into the supply airstream duct 116. A plurality of zone dampers D1 to Dn may be installed in the plurality of interconnected supply channel ducts such that one of the zone dampers D1 to Dn is configured in each of the supply channel ducts for individually regulating the flow of supply air into each zone 104 via the HVAC device 108. The central dampers Dc and the zone dampers D1 to Dn may facilitate in controlling the ventilation of the zones 104. The AHU 106 or each HVAC device 108 may include a cooling unit, a heating unit, a set of filters, a dehumidification unit, a fan assembly, and the like, which may facilitate in conditioning the quality of the air being supplied to the respective zones 104. The set of filters may facilitate controlling the degree of filtration of the air being supplied into the zone 104. The air being supplied (supply air) may include outside air OA, return air RA, or a combination thereof. The detailed operation of the HVAC system and the AHU 106 have been described later in conjunction with FIG. 2.

The server 114 or controller 112 may be configured to be operatively connected to the AHU 106, the dampers D, the HVAC devices 108, the IAQ sensors 110, and other components of the system 100 or BMS to control the operation of the BMS. The server 114 may include a processing device comprising one or more processors coupled to a memory device storing instructions executable by the processing device to enable the server 114 to perform one or more designated operations.

The server 114 or the controller 112 may actuate the components of the BMS or AHU for creating an artificial calibration environment or calibration baseline for the IAQ sensors in order to calibrate the IAQ sensors 110. In one or more embodiments, the controller 112 can actuate the BMS or AHU 106 to maximally treat and circulate the air at the zones 104, by increasing the ventilation, outdoor air percentage, dedicated outdoor air ventilation, degree of air filtration, and/or similar parameters at the zones 104 of the environment/AOI 102 to ensure a consistent calibration environment or a calibration baseline in the environment/AOI. Further, in one or more embodiments, to use this consistent environment as a calibration baseline, a reference sensor that may be a new, factory-calibrated sensor (not shown) may optionally be incorporated into the supply channel ducts 116, or the air as treated by the AHU 106 may be considered clean. The controller 112 may be configured to calibrate the IAQ sensors 110 by supplying maximally filtered/fresh air to both the reference sensor and the IAQ sensors 110 to be calibrated. The controller 112 may compare the IAQ value sensed by the IAQ sensor (to be calibrated) and the reference sensor in order to calibrate the IAQ sensors as both the reference sensor and the IAQ sensors share a common air supply.

Furthermore, as the environment/AOI 102 is made consistent, the IAQ sensors 110 in the environment/AOI 102 may be allowed to detect IAQ values of the calibrated environment and the IAQ sensors 110 may be accordingly calibrated by the BMS or controller 112 based on an average value of the IAQ values being detected by each of the IAQ sensors 110-1 to 110-N. The averaging method may be suited for IAQ sensors with random drift. However, other calibration techniques may also be employed once the environment/AOI is made consistent.

In one or more embodiments, the controller 112 may be configured to actuate the BMS/AHU 106 to maximally treat and circulate the air at the zones 104 to create a predetermined calibration environment for the IAQ sensors 110 of the AOI 102. The controller 112 may create the predetermined calibration environment by actuating the fan assembly, central damper, and zone dampers of the BMS/AHU 106 to perform one or more of adjusting inflow of outside air into the AOI 102 to a first predetermined maximum level, adjusting the ventilation rate of the AOI 102 to a second predetermined maximum level, and increasing a degree of air filtration in the AOI 102 to a third predetermined maximum level. Once the predetermined calibration environment is created, the controller 112 may monitor IAQ values detected by the IAQ sensors 110 at the AOI in the predetermined calibration environment and correspondingly calibrate the IAQ sensors 110 based on the monitored IAQ values being detected by each of the IAQ sensors 110. In one or more embodiments, the controller 112 may be configured to calibrate the IAQ sensors 110 based on an average value of the monitored IAQ values being detected by each of the IAQ sensors 110.

In one or more embodiments, the controller 112 may actuate the fan assembly, central damper Dc, and zone dampers D1 to Dn of the AHU or BMS to adjust the inflow of the outside air into the AOI 102 to the first predetermined maximum level, and/or further adjust the ventilation rate of the AOI 102 to the second predetermined maximum level. In addition, the controller 112 may actuate the AHU 106 or the air filters to increase the degree of air filtration in the AOI 102 to the third predetermined maximum level.

In one or more embodiments, the system may include one or more occupancy sensors 120-1 t0 120-N (collectively referred to as occupancy sensors 120, herein) positioned at zones 104-1 to 104-N of the AOI 102, which may be in communication with the controller 112. The occupancy sensors 120 may be operable to detect the presence of occupants at the zones 104/AOI 102. The controller 112 may be configured to create a predetermined calibration environment when no occupants are detected in the AOI 102 by the occupancy sensors 120. This may help eliminate any disturbance caused by occupants during the treatment and circulation of air at the AOI 102 while creating the calibration environment and further protect the occupants from any exposure to the calibrated environment during the process.

In one or more embodiments, the system may include one or more secondary sensors 122 comprising one or more of a temperature sensor, a humidity sensor, a volatile organic compound sensor, a carbon dioxide sensor, a particulate matter sensor, a carbon monoxide sensor, an ethylene sensor, a formaldehyde sensor, a radon sensor, a methane sensor, an ozone sensor, a sulfur dioxide sensor, a nitric oxide, and a nitrous oxide sensor, positioned outside the AOI 102 or in the channel ducts of the BMS/AHU, which may be in communication with the controller 112 of the BMS. The secondary sensors 122 may be operable to monitor the level of air qualityparameters, temperature, and humidity of the outside air OA or return air RA. The controller 112 may be configured to create the predetermined calibration environment based on the monitored level of air quality parameters, temperature, and humidity of the outside air OA. For exemple, based on whether the monitored levels are within predefined threshold values. For instance, if the IAQ of the environment/AOI 102 is detected to be better than the outside air, the controller 112 may restrict the inflow of the outside air while creating the calibration environment for the IAQ sensors 110, as low quality outside air may affect the creation of clean and treated calibration environment for the IAQ sensors 110. However, if the outside air is detected to be better than the IAQ of the environment/AOI 102 or if the outside air is detected to be above a safe level, the controller 112 may allow the inflow of the outside air for creating the calibration environment for the IAQ sensors 110. In one or more embodiments, air quality maybe considered better in one area (e.g., indoor area) if one or more of the monitored parameters, temperature, and/or humidity values in the area is lower than a corresponding one or more of the monitored parameters, temperature, and/or humidity values in another area (e.g., outdoor). In one or more embodiments, air quality maybe considered better in one area if an average of values monitored by sensors in the area is lower than an average of values monitored by sensors in another area.

In one or more embodiments, the system 100 and/or server 114, and/or controller 112 may be operatively coupled to a website and so be operable and monitorable from any Internet-enabled user device or mobile device. Examples of user devices or mobile may include but are not limited to, a portable computer, a personal digital assistant, a handheld device, and a workstation. The IAQ sensors 110 and the HVAC device 108 may be in communication with each other and with the server 114 or controller 112 of the BMS through the network 118 via wired and/or wireless media.

In one implementation, the network 118 can be a wireless network, a wired network or a combination thereof. Network 118 can be implemented as one of the different types of networks, such as intranet, local area network (LAN), wide area network (WAN), the internet, and the like. Further, the network 118 may either be a dedicated network or a shared network. The shared network represents an association of the different types of networks that use a variety of protocols, for example, Hypertext Transfer Protocol (HTTP), Transmission Control Protocol/Internet Protocol (TCP/IP), Wireless Application Protocol (WAP), and the like, to communicate with one another. Further, network 118 can include a variety of network devices, including transceivers, routers, bridges, servers, computing devices, storage devices, and the like. In another implementation the network 118 can be a cellular network or mobile communication network based on various technologies, including but not limited to, Global System 100 for Mobile (GSM), General Packet Radio Service (GPRS), Code Division Multiple Access (CDMA), LoRaWAN, Long Term Evolution (LTE), WiMAX, 5G or 6G network protocols, and the like.

Referring to FIG. 2, method 200 for creating an artificial calibration environment to calibrate the IAQ sensors of the multi-zone environment/AOI. Method 300 may involve the AHU 106, HVAC devices 108, IAQ sensors 110, server 114, controller 112, and other components associated with the existing BMS of the environment/AOI 102 or components of the system 100 of FIG. 1. Method 200 may include step 202 creating a predetermined calibration environment at the AOI by enabling the BMS associated with the AOI to perform one or more of adjusting inflow of outside air into the AOI to a first predetermined maximum level, adjusting ventilation rate of the AOI to a second predetermined maximum level, and increasing a degree of air filtration in the AOI to a third predetermined maximum level.

Method 200 may include step 204 of monitoring IAQ values detected by the one or more IAQ sensors at the AOI in the predetermined calibration environment. Further, method 200 may include step 206 of calibrating the IAQ sensors based on the monitored IAQ values being detected by each of the IAQ sensors. In one or more embodiments, step 206 includes calibrating the IAQ sensors based on an average value of the monitored IAQ values being detected by each of the IAQ sensors.

In one or more embodiments, step 202 may include actuating a central damper, and/or zone dampers associated with each of the zones and a fan assembly associated with the AHU to adjust the inflow of the outside air into the zones to the first predetermined maximum level and/or further adjust ventilation rate of the zones to the second predetermined maximum level. Further, step 202 may include actuating the air filter of AHU or an air filtration system associated with the BMS to increase the degree of air filtration in the AOI to the third predetermined maximum level.

In one or more embodiments, the calibration environment may be created when no occupants are present in the environment/AOI. Further, the calibration environment may be created at a predefined time comprising night time or when the outside air quality is better than predefined threshold values.

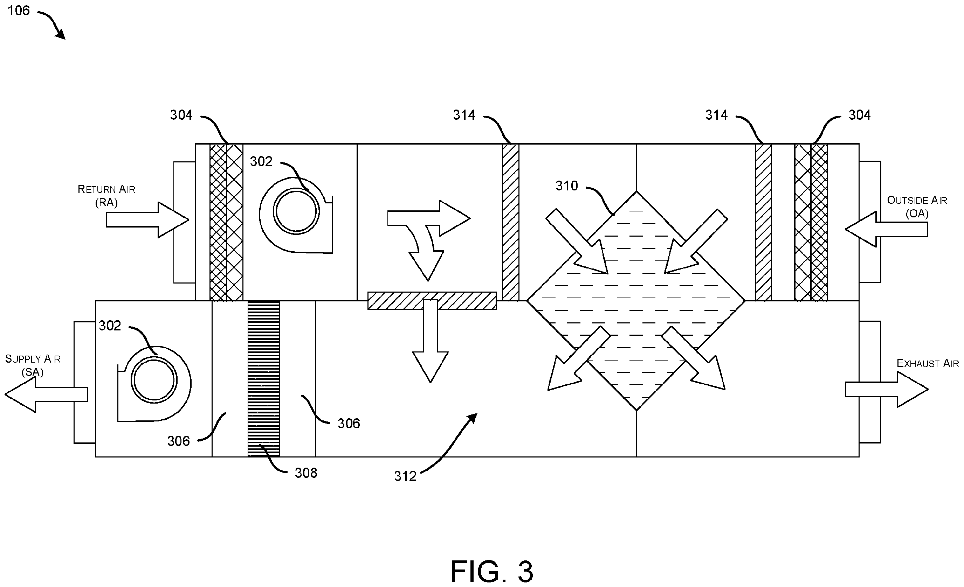

Referring to FIG. 3, in one or more embodiments, the air handling unit (AHU) 108 employed in system 100 and method 200 may be used to regulate and circulate air within the zones 104 of the environment/AOI 102. AHU 106 may be a part of a larger heating, ventilation, and air conditioning system of the AOI 102, which may be connected to the HVAC devices assigned to each zone 104. The AHU 106 is the composition of elements mounted in large, accessible box-shaped units installed at the AOI 102, which may be responsible for conditioning the supply air to ensure that the supply air is at the desired temperature, humidity, and air quality. The AHU 106 may include blowers/fan assembly 302, filters 304, heating or cooling coils 306, humidifiers or dehumidifiers 308, heat exchangers 310, a mixing chamber 312, and the like. The blower or fan assembly 302 may be responsible for moving air through the AHU 106 or into the zones, while the filter 304 may remove dust, pollen, and other contaminants from the outside air OA or return air RA. Filters 304 depending on the air purity requirements may be selected to provide a higher or lower particle, viruses, bacteria, odor, and other air pollutants retention. The heating or cooling coils 306 may be used to adjust the temperature of the supply air SA, while the humidifiers and dehumidifiers 308 control the moisture content of the air to be supplied to the zones 104. The heat exchanger device 310 may transfer temperature between two fluids, in this case, coolant and the air, separated by solid barriers.

The AHU 106 or BMS may manage proper ventilation at the zones 104 with the air and further perform other functions such as but not limited to filtration and control of the quality of the air that may reach the zones 104, and control of the air temperature that regulates the air conditioning system in cold or hot so that the IAQ values in the zones 104 is in a desirable or user-defined range. The BMS may further monitor and control the relative humidity for greater indoor comfort in the zones 104. It is to be appreciated that the same BMS of the environment/AOI may enable the creation of an artificial predictable calibration environment or baseline for the IAQ sensors 110 of the environment/AOI 102 for automatically calibrating the IAQ sensors 110 without any additional hardware components, thereby making the system 100 and method 200 simple, efficient, and cost-effective.

Thus, the invention provides a simple, efficient, and cost-effective system and method that involve the existing building management system of the multi-zone environment/AOI for creating an artificial calibration environment or baseline for the IAQ sensors to calibrate the IAQ sensors of the multi-zone environment/AOI without additional hardware components.

While the subject disclosure has been described with reference to exemplary embodiments, it will be understood by those skilled in the art that various changes may be made and equivalents may be substituted for elements thereof without departing from the scope of the claims. Modifications may be made to adopt a particular situation or material to the teachings of the subject disclosure without departing from the scope of the claims. Therefore, it is intended that the subject disclosure not be limited to the particular embodiment disclosed, but that the subject disclosure includes all embodiments falling within the scope of the claims.

In interpreting the specification, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refer to at least one of something selected from the group consisting of A, B, C ....and N, the text should be interpreted as requiring only one element from the group, not A plus N, or B plus N, etc.

## Claims

1. A method for calibrating indoor air quality sensors associated with an area of interest, the method comprising the steps of:
creating a predetermined calibration environment for one or more indoor air quality sensors associated with the area of interest;
monitoring indoor air quality values detected by the one or more indoor air quality sensors at the area of interest in the predetermined calibration environment at a predefined time; and
calibrating the one or more indoor air quality sensors based on the monitored indoor air quality values being detected by each of the indoor air quality sensors.

2. The method of claim 1, wherein the method comprises the step of calibrating the one or more indoor air quality sensors based on an average value of the monitored indoor air quality values being detected by each of the indoor air quality sensors.

3. The method of claim 1 or 2, wherein the method of creating the predetermined calibration environment comprises the steps of enabling a building management system associated with the area of interest to perform one or more of adjusting inflow of outside air into the area of interest to a first predetermined maximum level, adjusting ventilation rate of the area of interest to a second predetermined maximum level, and increasing a degree of air filtration in the area of interest to a third predetermined maximum level;
and wherein the method optionally further comprises the steps of:
actuating an air handling unit associated with the building management system to adjust inflow of the outside air into the area of interest to the first predetermined maximum level, and/or further adjust ventilation rate of the area of interest to the second predetermined maximum level; and
actuating the air handling unit or an air filtration system associated with the building management system to increase the degree of air filtration in the area of interest to the third predetermined maximum level.

4. The method of claim 3, wherein the area of interest is a multi-zone building comprising one or more zones, wherein the method of creating the calibration environment comprises the steps of:
actuating air dampers associated with each of the one or more zones and a fan assembly associated with the air handling unit to adjust inflow of the outside air into the one or more zones to the first predetermined maximum level, and/or further adjust ventilation rate of the one or more zones to the second predetermined maximum level; and
actuating the air handling unit or an air filtration system associated with the building management system to increase the degree of air filtration in the one or more zones to the third predetermined maximum level.

5. The method of any preceding claim, wherein the predetermined calibration environment is created:
when no occupant is detected in the area of interest;
during the predefined time comprising night time; and/or
when temperature, and humidity of the outside air and/or a level of air quality parameters comprising one or more of volatile organic compounds, carbon dioxide, particulate matter, carbon monoxide, ethylene, formaldehyde, radon, methane, ozone, sulfur dioxide, nitric oxide, oxygen, and nitrous oxide present in the outside air are within predefined threshold ranges, wherein data pertaining to the air quality parameters, the temperature, and the humidity of the outside air is received from one or more secondary sensors positioned outside the area
of interest and/or from a server; and
wherein the one or more secondary sensors optionally comprise one or more of a temperature sensor, a humidity sensor, a volatile organic compounds sensor, a carbon dioxide sensor, a particulate matter sensor, a carbon monoxide sensor, an ethylene sensor, a formaldehyde sensor, a radon sensor, a methane sensor, an ozone sensor, an oxygen sensor, a sulfur dioxide sensor, a nitric oxide, and a nitrous oxide sensor.

6. A system to calibrate indoor air quality sensors associated with an area of interest, the system comprising:
a controller associated with a building management system of the area of interest, wherein the controller is in communication with one or more indoor air quality sensors associated with the area of interest;
wherein the controller is configured to:
actuate the building management system to create a predetermined calibration environment for the one or more indoor air quality sensors of the area of interest;
monitor indoor air quality values detected by the one or more indoor air quality sensors at the area of interest in the predetermined calibration environment at a predefined time; and
calibrate the one or more indoor air quality sensors based on the monitored indoor air quality values being detected by each of the indoor air quality sensors.

7. The system of claim 6, wherein the controller is configured to calibrate the one or more indoor air quality sensors based on an average value of the monitored indoor air quality values being detected by each of the indoor air quality sensors.

8. The system of claim 6 or 7, wherein the controller is configured to create the predetermined calibration environment by actuating the building management system to perform one or more of adjusting inflow of outside air into the area of interest to a first predetermined maximum level, adjusting ventilation rate of the area of interest to a second predetermined maximum level, and increasing a degree of air filtration in the area of interest to a third predetermined maximum level; and
wherein the controller is optionally configured to:
actuate an air handling unit associated with the building management system to adjust inflow of the outside air into the area of interest to the first predetermined maximum level, and/or further adjust ventilation rate of the area of interest to the second predetermined maximum level; and
actuate the air handling unit or an air filtration system associated with the building management system to increase the degree of air filtration in the area of interest to the third predetermined maximum level.

9. The system of claim 6, 7 or 8, wherein the system comprises one or more occupancy sensors positioned at the area of interest and in communication with the controller, the one or more occupancy sensors are operable to detect presence of occupants at the area of interest,
wherein the controller is configured to create the predetermined calibration environment when no occupants are detected in the area of interest by the one or more occupancy sensors.

10. The system of any of claims 6-9, wherein the system comprises one or more secondary sensors comprising one or more of a temperature sensor, a humidity sensor, a volatile organic compound sensor, a carbon dioxide sensor, a particulate matter sensor, a carbon monoxide sensor, an ethylene sensor, a formaldehyde sensor, a radon sensor, a methane sensor, an ozone sensor, a sulfur dioxide sensor, a nitric oxide sensor, an oxygen sensor, and a nitrous oxide sensor positioned outside the area of interest and in communication with the controller, the one or more secondary sensors are operable to temperature, and humidity of the outside air and/or a level of air quality parameters comprising one or more of volatile organic compounds, carbon dioxide, particulate matter, carbon monoxide, ethylene, formaldehyde, radon, methane, ozone, sulfur dioxide, nitric oxide, oxygen, and nitrous oxide present in the outside air,
wherein the controller is configured to create the predetermined calibration environment when the monitored level of the air quality parameters, the temperature, and the humidity of the outside air is detected to be within predefined threshold values.

11. The system of any of claims 6-10, wherein the area of interest is a multi-zone building comprising one or more zones.

12. The system of any of claims 6-11, wherein the one or more indoor air quality sensors comprise one or more of a volatile organic compound sensor, a carbon dioxide sensor, a particulate matter sensor, a carbon monoxide sensor, an ethylene sensor, a formaldehyde sensor, a radon sensor, a methane sensor, an oxygen sensor, an ozone sensor, a sulfur dioxide sensor, a nitric oxide, and a nitrous oxide sensor.

13. A method for creating a calibration environment for indoor air quality sensors associated with an area of interest, the method comprising the steps of:
enabling a building management system associated with the area of interest to perform one or more of adjusting inflow of outdoor air into the area of interest to a first predetermined maximum level, adjusting ventilation rate of the area of interest a second predetermined maximum level, and increasing a degree of air filtration in the AOI to a third predetermined maximum level.

14. The method of claim 13, wherein the calibration environment is created at a predefined time comprising night time and/or when no occupant is detected at the area of interest.

15. The method of claim 13 or 14, wherein the calibration environment is created when air quality of the outside air is less than predefined threshold values.
